Europäisches Patentamt

European Patent Office

Office européen des brevets

(11) Publication number: **0 204 807**
**B1**

(12) EUROPEAN PATENT SPECIFICATION

(45) Date of publication of patent specification: 02.05.90

(21) Application number: 86900317.8

(22) Date of filing: 05.12.85

(86) International application number:
PCT/SE85/00504

(87) International publication number:
WO 86/03589 19.06.86 Gazette 86/13

(51) Int. Cl.⁵: **G 01 N 33/543**

(54) METHOD, APPARATUS AND SYSTEM FOR CONDUCTING BIOSPECIFIC AFFINITY ASSAY INVOLVING COLUMN WITH REFERENCE PORTION.

(30) Priority: 07.12.84 SE 8406219

(43) Date of publication of application:
17.12.86 Bulletin 86/51

(45) Publication of the grant of the patent:
02.05.90 Bulletin 90/18

(84) Designated Contracting States:
AT BE CH DE FR GB IT LI LU NL SE

(56) References cited:
EP-A-0 060 700
EP-A-0 093 613
US-A-3 451 777
US-A-4 294 817
US-A-4 366 241

(73) Proprietor: PHARMACIA AB
Rapsgatan 7
S-751 82 Uppsala (SE)

(72) Inventor: SCHRÖDER, Hasse
Grabergsvägen 2
S-752 40 Uppsala (SE)
Inventor: HENDMONT, Hans, Gunnar
Brodyrvägen 22
S-752 57 Uppsala (SE)
Inventor: KOBER, Anita
Petterslundsgatan 15
S-753 28 Uppsala (SE)

(74) Representative: Widén, Björn et al
UPPSALA PATENTBYRA P.O. Box 9013
S-750 09 Uppsala (SE)

Courier Press, Leamington Spa, England.

## Description

The present invention relates to a method for conducting analyses or assays based upon biospecific affinity reactions, particularly multi-step assays, in a simplified and more rapid way than before but with maintained reliability. The invention also relates to an apparatus and a system therefor.

A great part of the biochemical tests which now are carried out in clinical laboratories are based upon biospecific reactions, particularly immunological reactions, i.e. so-called immunoassays. These tests are generally so complicated and time-consuming that in practice they may only be conducted in special laboratories. In many situations it would, however, be desirable to be able to quickly conduct such assays in an ordinary doctor's office. Attempts have been made to simplify the test methods into so-called "doctor's office tests", but no such analytical methods and systems have yet been developed that are sufficiently simple, well functioning and reliable. There is therefore a need of simplified methods and systems which permit semi-quantitative or quantitative biochemical assays of the above mentioned kind to be conducted quickly and in a simple way in an ordinary doctor's office or in the field.

The invention relates to such a simplified method as well as to an apparatus and a system for carrying out the method, which method, apparatus and system have the features stated in the accompanying claims and are described in more detail hereinafter.

The method, apparatus and system of the present invention are intended for such analyses or assays wherein one or more substances in a fluid sample, e.g. from blood serum, are determined by means of one or more biospecific affinity reactions, the substance or substances to be determined being bound to carrier immobilized receptors for the respective substance to form immobilized complexes, which are then, depending on the particular analytical system used, determined either directly, or after a developing reaction with an indicator component or marker giving a detectable product, visually or by instrumental means, such as photometrically, e.g. colorimetrically, fluorimetrically etc.

The term receptor as it is used in the present specification and claims means a compound (structure) exhibiting a biospecific affinity to another compound (structure), usually called a ligand. By definition the terms receptor and ligand are interchangeable with each other, such that a ligand is an example of a receptor, and *vice versa.*

As the carrier for the above mentioned receptors the prior art methods of analysis have usually utilized small gel or cellulose spheres, polysaccharide beads/particles, small paper discs, plastic surfaces or simply the inner side of a test tube. The previous methods have several disadvantages. Inter alia, many of the methods require separation steps, e.g. centrifugation operations, which introduce sources of error and may make additional apparatus necessary, which normally is not available in an ordinary doctor's office. In case an active standard has been used in the measurement procedure, such a standard has usually been treated separately in a corresponding way as the sample. Exemplary of prior art methods are those disclosed in EP—A1—60700, EP—A1—93613, US—A—3,451,777 and US—A—4,294,817.

According to the method of the present invention the measurements of both the sample and the standard are performed in one and the same reaction vessel but in different zones thereof. To this end the reaction vessel is comprised of a column containing a flow transmitting carrier material, preferably a particulate material, the filled part of said column being divided into a test portion, wherein one or more receptors for the analyte, i.e. the substance to be determined, are bound to the carrier material, and a reference portion (or a standard zone) containing the same or another carrier material and adapted to provide a reference for the substance or substances to be determined, said test and reference portions being arranged either in a superposed relationship in the flow direction or in a parallel relationship. To carry out the necessary reaction steps the respective analytical solutions, i.e. the sample solution and the necessary reagent and washing solutions, respectively, are drawn sequentially, and when necessary with intervening incubation intervals, into the column, such that a subsequent addition of solution displaces the preceding one, whereupon the determination of the substance or substances in question is performed by a comparative measurement of the above mentioned test and reference portions directly on the column.

The term "column", as it is used in the present specification and claims, is to be interpreted in a broad sense and means any container or body member that may be used for the purposes of the invention irrespective of the shape thereof. Thus, in addition to cylinder-shaped elements, e.g., also square or rectangular cross-sectional elements of the measuring cell type, are included.

The above mentioned reference portion may either contain a fixed standard, e.g. a known or predetermined amount of an indicator or a marker substance, or it may be adapted to provide said standard during the assay, e.g. by coupling to said column material a known quantity of the substance to be determined, or of a substance reacting in the same way with the reagent in question (indicator or marker). Thus, in, for example, an immunological assay said substance should have at least one determinant or epitope in common with the test substance. If it is desired to determine the mutual relationship or ratio of two components of a sample, said test portion of the column may be adapted to bind to one component thereof and the reference portion to bind to the other component.

Based upon the above described concept an apparatus for conducting the assays may be constructed, which apparatus according to the present invention comprises

a) a column member having an inlet opening at one end thereof and containing a carrier material, preferably in the form of particles, which carrier material is divided into at least one test portion, to which one or more receptors for the substance or substances to be determined are bound, and at least one reference portion (of the same or another carrier material) adapted to provide a reference or standard for the substance or substances to be determined, said test and reference portions being arranged either in a superposed relationship in the flow direction or in a parallel relationship,

b) an aspirator and fluid collecting device connected or connectable to one end of the column member and comprising a hollow member having an axially movable sealing plunger means therein, which plunger means preferably is manually actuable, at least part of said column member being transparent to permit a comparative visual or instrumental determination of the material of said test and reference portions directly on the column.

In a simple and advantageous embodiment of the apparatus of the invention the aspirator and fluid collecting device thereof may consist of the plunger/cylinder part of an injection syringe of a *per se* known type, which is arranged to be attached to the column member in any suitable manner, e.g., with a snap lock. A stop means may optionally be provided for the plunger means to only permit movements thereof in the aspirating direction. Appropriate markings for the desired volumes of the various analytical solutions for a particular assay may, for example, be provided on the syringe cylinder.

To facilitate the manual operation of such an apparatus at least the syringe part thereof may be supported in a handle or an adapter having a suitable actuator means, e.g. readily accessible to the thumb, in operative engagement with the plunger and by which the syringe plunger may be moved in a more controlled manner, such as, e.g., a gear wheel/rack transmission. A gear unit may optionally be provided to reduce the plunger movement obtained by the transmission of a certain thumb movement thereto, thus permitting smaller plunger movements and thereby a greater accuracy. The handle or adapter may also be arranged to give off a snapping sound or the like, when the required volumes of sample, washing solution and reagents, respectively have been achieved. Instead of the above mentioned gear wheel/rack arrangement any other suitable drive means, such as, e.g. a biased spring device, may, of course, also be used.

The determination or measurement, of the test and/or reference portions of the column may, as mentioned above, be effected visually or instrumentally, e.g. by photometric means. In the latter case, i.a. depending on the particular immobilized complex formed and/or the particular indicator substance used, (i.e. whether a colour, fluorescent, phosphorescent, luminescent, etc. system is obtained) any suitable instrument of a *per se* known type may be used such as colorimeters, spectrophotometers, fluorimeters, phosphorimeters, etc., which, however, should be suitably adapted to the respective measurement on said column member.

A system for conducting the intended assays thus may comprise, in addition to said apparatus, photometric measuring means as above. Such a system may conveniently be automated by also providing plunger drive means for operative engagement with the plunger means of said apparatus, fluid distribution means to be connected to the column member of said apparatus for dispensing the analytical solutions, and control means, such as a computer means, for controlling said system components.

Examples of tests, for which the method and apparatus of the invention may be used, comprise those based upon immunological reactions, such as the reaction of antigen (or hapten) with antibody, and other biospecific affinity reactions based upon other receptor-ligand interactions, e.g., enzyme reactions, such as enzyme-substrate or enzyme-coenzyme, lectin-sugar, protein A-Fc-part of IgG, biotin-avidin, neurotransmitter/receptor reactions, such as acetylcholine-line receptor, etc. The tests may in conventional manner be performed non-competitively as well as competitively.

The invention is particularly well suited for multi-step analytical systems, e.g. of the so-called sandwich type, i.a. wherein the substance (ligand) to be determined is bound biospecifically between two reactants (receptors). This is particularly the case for various immunoassays based upon said sandwich principle. For example, such an immunoassay may readily be conducted, e.g. on blood plasma, with an apparatus according to the invention, wherein the column member contains a suitable carrier material, which as described above in a portion thereof in *per se* known manner supports a receptor for a substance to be determined in the sample, and in another part of the column supports a known quantity of said substance to be determined or of a substance having a corresponding reaction specificity, such as discussed above, intended to be used as a standard in the measuring step. The carrier material of said two column portions are preferably separated by a suitable filter or by a layer of carrier material without any immobilized reactant. In operation a suitable sample amount is aspirated into the column (according to a marking on the syringe cylinder or in any other suitable way) and is allowed to incubate for a sufficient period of time. A proper amount of washing solution is then aspirated into the column to displace the sample solution, followed by a sufficient amount of indicator solution, e.g., containing a reactant of a suitable biospecific affinity and provided with, for example, a chromophoric, fluorescent, chemiluminescent or enzymatically active group, e.g., an enzyme conjugate of an enzyme with an antibody capable of binding to the substance to be determined. After a possible incubation for the indicator reagent to bind sufficiently, on one hand, to the sample substance which has been attached to the receptor of the column test portion and, on the other hand, to the predetermined amount of said substance, or analogue thereto, immobilized in the reference portion of the column material, a suitable amount of washing solution is again aspirated to displace the indicator reagent solution. In the case of enzyme reagents for fluorimetry, for example, a substrate solution is first aspirated which by the action of the enzyme produces a fluorescent product, whereupon the fluorescent activity of the test and reference portions of the column are determined by measurements directly on the column by means of a suitably adapted fluorimeter and the values obtained are compared whereby the contents of said substance in the sample may be calculated. If desired, the sample and indicator reagents may at least for certain combinations thereof be mixed with each other before being drawn into the column.

In many tests it is only interesting to know if the concentration of a certain substance of a sample is above or below a certain level, and a quantity of said substance, or analogue thereto, which gives a signal corresponding to said level in the measuring step, is then selected as said standard amount in the reference portion of the column.

From the above it appears that the method and the apparatus of the present invention provide an analytical means, which is simple and quick to perform with a minimum risk of mistakes by the operator and which consequently may be readily performed by a doctor or a nurse in an ordinary doctor's office. Due to the fact that all the aspirated solutions are retained in the syringe cylinder during the whole procedure also the "splashing" with solutions is minimized. Further, the feature of aspirating the sample into a column provides an advantageous concentrating effect of the substance to be determined.

Semi-quantitative as well as quantitative analyses or assays may well be carried out with the present method and apparatus. In addition the measuring device, i.e. a photometer, such as a fluorimeter, etc., which may be necessary may have a relatively simple construction and may therefore be made inexpensive. Preferably, the measuring device is integrated with computing means, such as a microcomputer, for quantification of the test results, which, e.g., may be presented on a display. For such semi-quantitative analyses where it is only desired to know whether the contents of a substance in a simple is above or below a certain level, one or more light signal generating devices are preferably provided, which light to indicate a positive and/or negative reply to the analysis.

If desired, the method and the apparatus according to the invention permit several substances to be determined simultaneously by dividing the test portion of the column into several parts for different receptors, and, when necessary, dividing the reference portion of the column into a corresponding number of portions having different standards.

The choice of carrier material for the column is not critical *per se*. The carrier material must, however, provide a large contact surface, give a low background signal, have a high capacity for covalent coupling of receptor and provide good flow properties. Suitable carriers are in many cases those used for affinity chromatography. Preferably, the material characteristics are such that substantially diffusion independence is obtained for the different reactions. For a more detailed discussion of such diffusion independence it is referred to, e.g., our PCT publication WO 84/03150.

The support material should be porous and/or particulate and may, e.g., be selected from those based upon dextran, polyacrylamide, polystyrene and glass, e.g., particulate agarose. A suitable particle size for particulate material is 1—200 micrometers, preferably 20—150 micrometers.

The method and the apparatus of the invention are, for example, excellently suitable for a rapid or screen test of allergies. As is well known hypersensitiveness to a certain allergen of the blood produces antibodies of the IgE type which are specific to the allergen. The well-known Phadebas RAST® test (Pharmacia AB, Sweden) is based upon this phenomenon. With said test one may directly from a blood sample taken from a patient determine whether the patient is allergic to specific substances. The principle of the Phadebas RAST® test is the same as for a conventional immunoassay of the sandwich type. A serum sample from the patient is contacted with a paper piece to which an allergen is coupled. Hereby only those allergens are bound which are specifically directed against the allergen in question. The other IgE molecules of the sample are removed by decanting and washing, and radioactively labelled antibodies against IgE are then added to the paper piece. The antibodies are bound in proportion to the serum sample amount of IgE which is specific to the allergen. By measuring the radioactivity of the paper piece a direct answer is obtained indicating if a patient has specific IgE against the allergen coupled to the paper piece. Instead of the radioactively labelled antibodies an enzyme conjugate of an enzyme with an antibody may be used, "developing" being effected by means of a substrate which by the action of the enzyme produces e.g. a colorimetrically or fluorimetrically detectable product. This conventional test is relatively complex and time-consuming and may therefore only be conducted in special laboratories.

A corresponding screen test to rapidly determine whether a patient has an IgE-mediated allergy may conveniently be performed with the method and the apparatus according to the invention. For this purpose a column is used whose test portion has a suitable allergen mixture, e.g., containing 5—15 different allergens, coupled to the carrier material, while a known amount of IgE is coupled to the carrier material of the reference portion of the column. The amount of IgE coupled to the reference portion is in this case selected such that when carrying out the method the signal obtained from the reference portion corresponds to the so-called "cut-off" value, which has been set statistically to catch as many allergic subjects as possible without too many false positive results. If the tested sample corresponds to allergy the amount of IgE taken up by the test portion will cause a signal which differs from that produced in the reference portion. Then one proceeds in the same way as described above to conduct an immunoassay according to the invention, i.e. by successively aspirating, and when necessary incubating, whole blood or plasma from a blood sample from the patient, reagent solution, washing solution, and, depending on the indicator, possibly a developing substrate solution. A measurement is then performed directly on the column, visually or with a suitable photometric means, such as a fluorimeter or colorimeter, to determine the indicator signal of the column test portion in relation to that of the column reference portion. If the result is positive, it means that the patient is allergic to one or more of the allergens. A more definite determination of the specific allergens to which the patient is sensitive may be performed in a corresponding manner with another column(s) having a reduced selection of allergens coupled thereto or in a special laboratory.

To determine whether a condition of a patient depends at all on an allergy a determination of total IgE may be effected by proceeding in the same way as above but, instead of the allergen mixture, coupling antibodies against IgE to the carrier of the column test portion. Such a determination of total IgE may optionally take place simultaneously with the test for allergen specific IgE by utilizing a three zones column having an allergen zone, an anti-IgE zone and an IgE zone.

If desired, it is, of course, also possible to test with respect to several such allergen mixtures at the same time or with respect to individual allergens by, as mentioned above, dividing the test portion of the column into several separate parts. In addition to achieving such a division through separate superposed carrier material layers the carrier material may also, as mentioned above, in per se known manner be divided into separated parallel segments.

As examples of other tests in the medical and other fields for which the method and apparatus of the invention are excellently suited may be mentioned the determination of IgG, IgM, IgA, serum proteins, glycoproteins and myoglobin. Further examples of substances which may be determined are theophyllin, digoxin and glycosylated hemoglobin. In the last-mentioned case it is usually the ratio of glycosylated to non-glycosylated hemoglobin that is of interest. Such a determination may, e.g., be performed by using a borate-group-containing material in the test portion of the column for taking up glycosylated hemoglobin and an ion-exchange material in the reference portion thereof to take up hemoglobin. After said uptake in the respective column portions the desired ratio may be obtained by, for example, a colorimetric comparison between the respective column layers.

In microbiological contexts the invention may, e.g., be used to determine the presence of various bacteria antigens in, for example, samples of various body fluids, but also antibodies directed against such antigens may be determined.

Hereinafter particular embodiments of the apparatus according to the invention, to which it, however, is not restricted in any way, will be described with reference to the accompanying drawings, wherein

Fig. 1 is a side-elevational view, partially in section, of an embodiment of an apparatus according to the invention,

Fig. 2 is a longitudinal sectional view of the column part of the apparatus of Fig. 1,

Fig. 3 is a side-elevational view of the adapter part of the apparatus of Fig. 1,

Fig. 4 is a bottom view of the adapter part of Fig. 3,

Fig. 5 is a perspective view of a measuring device adapted for use in the method according to the invention,

Fig. 6 is a schematic representation of an automated system according to the invention,

Fig. 7 is a side-elevational view, partially in section, of a modified column end part, adapted to the automated system of Fig. 6,

Fig. 8 is a graph showing the sample/reference signal ratio plotted against allergen specific IgE concentration of a sample, and

Fig. 9 is a corresponding graph as Fig. 8 for total IgE.

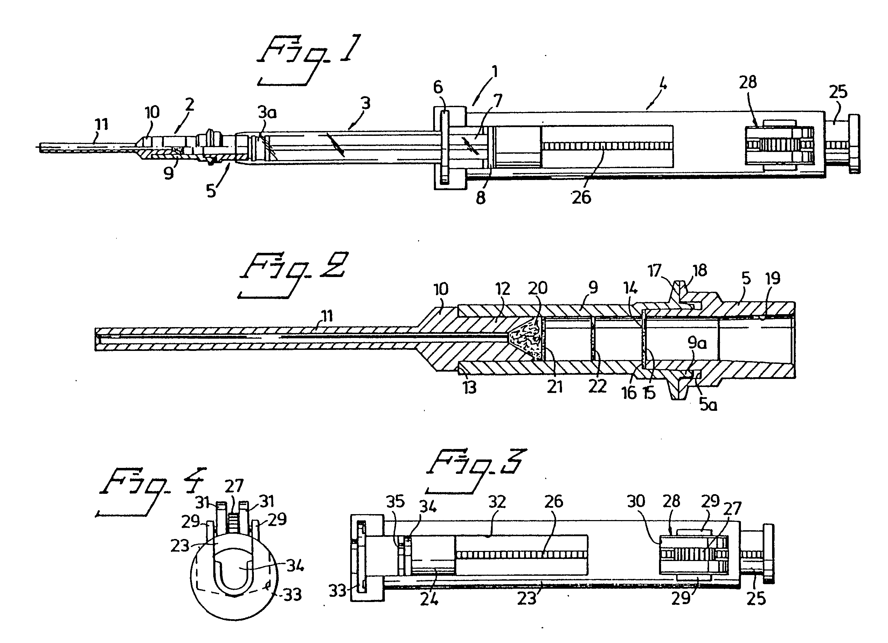

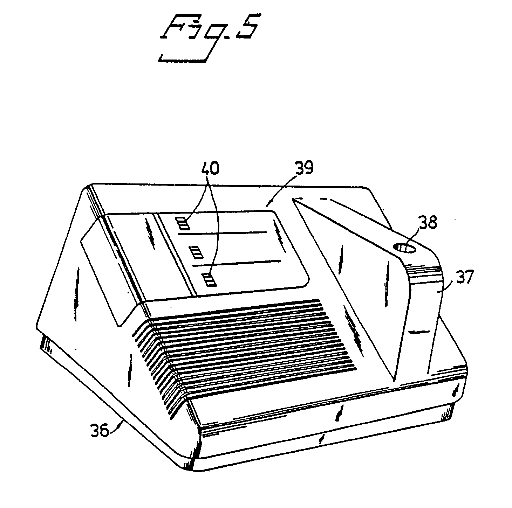

The apparatus of Fig. 1, generally designated by the reference numeral 1, comprises a column member 2 provided with a cannula and connected to a syringe member 3 supported in a manually manoeuvrable adapter or "pipette" 4. The column member 2, which will be described in more detail below in connection with Fig. 2, is filled with a carrier material (not shown). The syringe member 3 may suitably consist of the plunger/cylinder part of a conventional injection syringe and is removably attached to the top end or end part 5 of the column member by press fitting. The top end of the syringe 3 is fixed in the adapter 4, via the support flange 6 of the syringe cylinder and the operating flange 8 of the plunger 7, as will be described in more detail in connection with Fig. 3.

Fig. 2 shows the design of the column member 2 of Fig. 1 in more detail. As shown it consists of a column tube 9, to the end of which a top part 10 provided with a cannular tube 11 is removably attached. To this end the top part 10 is provided with an end section 12 having substantially the same diameter as the inner diameter of the column tube 9 and arranged to be fixed therein by press fitting, a shoulder portion 13 thereof abutting the end of the column member 9. In the upper part (i.e. the right part in Fig. 2) of the top piece 10 the bore of the cannular tube 11 widens continuously to provide a smooth transition to the inner wall of the column tube 9. The other end section of the column tube 9 has a larger inner diameter than the main part of the tube to receive a filter 14, e.g. of polypropylene or filter paper, and optionally a net 15, e.g. of nylon. The filter and net, respectively, are retained in position against the shoulder portion 16, provided by said diameter change, by means of the above mentioned end piece 5, whose outer diameter of the corresponding end portion is adapted to permit the fitting thereof into the column tube. Securing of the end piece 5 is accomplished by snapping the free tube edge 9a, extending beyond a flange portion 17 of the column tube 9, into a corresponding groove 5a in the flanged end portion 18 of the end piece 5 such that the opposed flanges abut each other. As mentioned above the end piece 5 is adapted to receive and fix the syringe 3 of Fig. 1, which, e.g., is an ordinary disposable hypodermic syringe, by pressing the cannula attachment part thereof into the slightly cone-shaped central bore 19 and snapping it fast. To improve the flow configuration in the transition between the tube 11 and the column tube 9, a plug 20 of a very porous material, e.g. Vyon® (Porvir Ltd, U.K.), is preferably provided and restricted by means of a filter 21, e.g. of polypropylene, filter paper, etc. Between the filters 21 and 14 the column material to be used in the device is packed and divided into two layers (not shown for clarity) separated by another filter 22, e.g., also of polypropylene or filter paper, as will be further described below. To permit a direct visual or instrumental, e.g. colorimetric or fluorimetric, determination on the column material in accordance with the invention, the column tube 9 is made of a transparent material. The preferably transparent cylinder of the syringe 3 is provided with appropriate volume markings (not shown) for the various analytical fluids of the particular analytical system used as will be described in more detail below.

As best appears from Fig. 3 and 4 the adapter 4 consists of a cylindrical casing 23, in which a plunger member 24 attached to an operating rod 25 is slidably mounted. The operating rod 25 is provided with a longitudinal, central rack 26, which in the top part (i.e. to the right in Fig. 3) of the adapter 4 engages a gear wheel 27 integral with a thumb wheel 28 (Fig. 4) and designed to be rotated by the thumb. The thumb wheel 28, and thereby the gear wheel 27, are rotatably mounted in two bracket members 29 projecting on the outside of the adapter 4 and extend through an upper recess or opening 30 into engagement with the rack 26. As appears from Fig. 4 the thumb wheel 28 consists of two wheel portions 31, arranged on either side of the gear wheel 27 to straddle the rack 26 and thereby simultaneously provide a guide means for the operating rod 25.

By moving the thumb wheel 28 the plunger member 24 may thus be moved along the adapter. A lower recess 32 of the adapter casing 23 extends up to the bottom end (i.e. the left end in Fig. 3) of the adapter 4 and is designed to permit the above mentioned insertion and fixation of the top of the syringe 3 in the adapter 4, the casing 23 thereof being provided with a transverse groove 33, adapted to receive the support flange 6 of the syringe cylinder. A corresponding groove 34 for the operating flange 8 of the syringe is provided in the lower part of the plunger member 24 as well as a recess 35 for the syringe cylinder. Due to said fixation of the syringe cylinder in the adapter casing 23, and of the syringe plunger rod 7 in the movable plunger member 24 of the adapter, any movement of the thumb wheel 28 will directly be transferred to the plunger 3a of the syringe 3. To increase the accuracy of the operation of the syringe plunger the adapter 4 may in a sophisticated variation thereof be provided with a transmission gear such that the plunger movement obtained when moving the thumb wheel 28 is transferred to the syringe plunger 3a. Further, if desired, the adapter 4 may be provided with a releasable lock means which only permits movement of the plunger member 24 in the aspiration direction of the syringe plunger.

The measuring device illustrated in Fig. 5, generally designated by 36, may be any suitable photometric means, such as a spectrophotometer, a fluorimeter or a colorimeter, depending on the possible marker used and thereby the wavelength selected for the measurement. (The term photometric means as used herein is meant to comprise any instrument for the measurement of selective absorption or emission of radiation, such as colorimeters, spectrophotometers, fluorimeters, phosphorimeters and luminescence meters). The device 36 comprises a cuvette section 37 having a vertical cylindrical measuring chamber 38 designed to receive the column tube 9 of the device 1 (Fig. 1). 39 designates an operating and reading section. The measuring device 36 may be of a conventional type having, for example, a dual beam path for simultaneous measurement of the test and reference portions, respectively, of the column. Preferably a microcomputer for processing the test data is included. The presentation of the results may be effected on a display, or, if it is only desired to find out whether the contents of a substance in a sample is above or below a certain level, as in the illustrated case by means of suitable lamps or light emitting diodes 40.

To conduct an allergy test with the apparatus and system of the invention shown in Figs. 1—5 one may proceed as follows:

The column tube 9 is first packed in a *per se* known manner with, on one hand, a carrier material, e.g. Sepharose® (Pharmacia AB, Sweden), having an allergen mixture, which, e.g., may contain up to 15 different allergens, coupled thereto, and, on the other hand, separated by means of the filter 22 in Fig. 2, the same carrier material to which IgE has been coupled. Then the column member 2 is fixed to the syringe 3, which, e.g., may be a disposable type syringe having a capacity of, for example, 2.5—5 ml, which in turn is engaged in the adapter 4, as described above, with the plunger 3a of the syringe 3 in its initial position, as will be described below. The apparatus 1 is then ready for use.

A suitable reagent set for the assay may, for example, be provided in a blister frame. The reagent solutions may for the particular assay be, in the order of use, transport buffer, washing solution, enzyme reagent solution (provided that an enzyme marker is used), washing solution and substrate solution.

A blood sample is taken from a patient, capillary or venous, e.g., 0.3—1 ml, whereupon the blood is transferred into a centrifuge tube and centrifuged at a suitable speed during, e.g.. about 1 minute. Alternatively, the blood is only filtered prior to use in the assay.

The syringe 3 is initially filled with a certain volume of transport buffer, e.g., about 1 ml. The apparatus is therefore first set in zero position by rotating the thumb wheel 28 of the adapter 4 until the plunger 3a of the syringe 3 has reached its bottom position. Hereby the system is "degassed" before the actual test procedure will be effected. Optionally, the adapter 4 may, as mentioned above, be arranged such that a snapping sound or the like also indicates that the required liquid volume has been achieved. Then blood plasma is carefully aspirated from the centrifuge tube, without disturbing the layer of red blood cells, by rotating the thumb wheel 28 until the syringe plunger reaches the next marking on the syringe cylinder. Washing solution is then aspirated by rotating the thumb wheel further and thereby moving the syringe plunger 3a to the marking indicating the correct volume. Reagent solution is then aspirated in the same way, and the whole device 1 is then placed into the holder means of the measuring device 36 and incubated for a suitable period of time, e.g., about 15—30 minutes.

After said incubation washing solution is again aspirated through the column by rotating the thumb wheel 28, and the necessary volume of substrate solution is then aspirated into the column. After possible incubation the apparatus 1 including the column tube 9 is placed into the recess 38 of the cuvette portion 37 of the measuring device. The measuring device 36 is then activated for measurement, whereupon a positive or negative result may be read on the reading or display section 39 thereof.

For an allergy test as described above the standard amount of IgE in the column is selected to a suitable "cut off" level corresponding to a clinically well documented IgE level for screening.

Suitably also a test for total IgE is conducted by proceeding in the same way as above, but replacing the allergen mixture coupled to the carrier material by antibodies against IgE.

For analyses or assays where the substances to be determined is present in relatively small concentrations a syringe 3 of greater capacity may be used to obtain a greater concentrating effect when adsorbing the substance in question onto the column carrier material.

It will be appreciated that the above described measuring system easily may be automated, utilizing the microcomputer of the measuring device for the control, by providing means for driving the plunger of the syringe 3 and connecting the cannular tube 11 by means of a suitable tube and valve system to the various analytical fluids concerned.

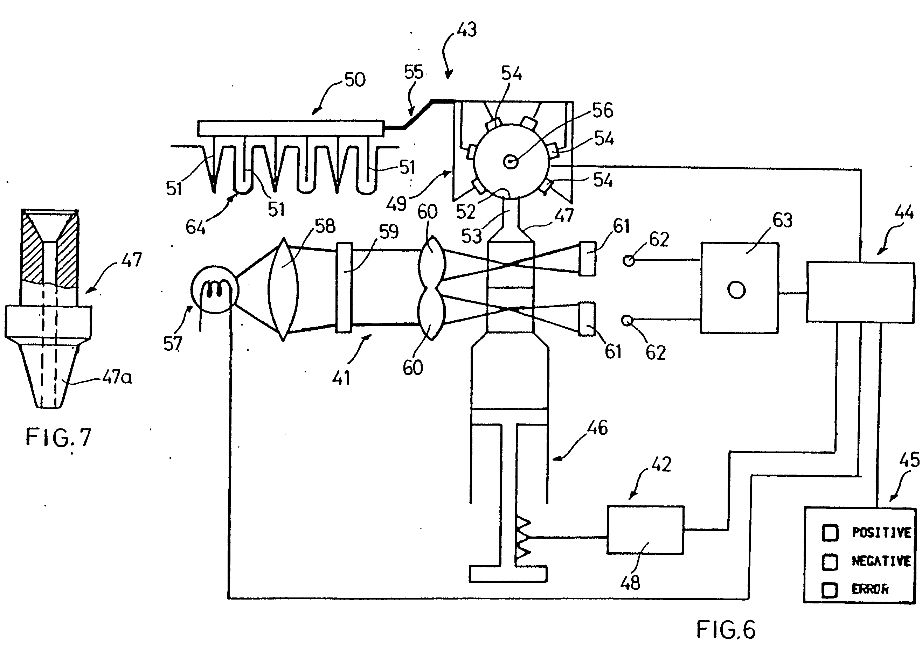

An example of such an automated system will be described hereinafter with reference to Figures 6 and 7. The system schematically represented in Fig. 6 comprises a fluorimeter unit 41, a plunger drive unit 42, a solution distribution means 43, a microcomputer 44 and and display section 45.

For use in this automated system no adapter is required for the column/syringe assembly, designated by the numeral 46 in Fig. 6. Further, the top or end piece 47 of the column part thereof is modified with respect to that of Fig. 1—4 in that the cannular part 11 of the column of Fig. 2 has been replaced by a short tapering portion 47a to provide a tubing mounting for connection to said solution distribution means 43. Suitable means are provided for receiving the column/syringe assembly 46 to position and retain the column thereof in the beam path of the fluorimeter unit 41, i.e. in the measuring chamber or "cuvette" portion thereof.

The plunger drive unit 42 comprises a suitable movable holder means for the syringe 46 for engaging the plunger rod thereof and operatively connected to a step motor 48. The latter is connected to the microcomputer 44 to be controlled thereby for effecting plunger movements corresponding to the aspiration of desired preset solution volumes into the column.

The solution distribution means 43 comprise a valve 49 and a comb-like member 50 having a plurality of cannulae 51, in the illustrated case six. The main port 52 of the valve 43 is connected to said modified top piece 47 of the column (Fig. 7), such as by tubing 53. In the illustrated case the main port 52 may be communicated with eight different ports. Six of these ports, designated by the numeral 54, are connected to a respective cannula 51 of the comb-like member 50 by tubing 55. The seventh valve port 56 is utilized for intake of air, and the eighth port is connected to the rear side of the apparatus and may be used as a drain. Suitable actuator means for the valve 43 are connected to the microcomputer 44 to be controlled thereby for connecting the column port 52 with the several ports 54 according to a preset schedule for the particular assay to be conducted.

The fluorimeter unit 41, which is of the dual beam path type with the beam paths aligned with the column test and reference portions, respectively, comprises a suitable light source 57, such as a halogen lamp, a monochromatic lens 58, an excitation filter 59 and two convergent lenses 60, all arranged in line transversely to the column/syringe assembly 46 on the fluorimeter excitation side. On the emission side of the fluorimeter (perpendicularly to the excitation beam path) there are provided a pair of emission filters 61 and a pair of detectors 62, such as light-sensitive diodes, to detect the radiation emitted by the column when irradiated with the excitation light from the light source 57. The detectors 62 are connected to a preamplification circuit 63, which together with said lamp source 57 are connected to the microcomputer 44.

In use, whe operator of the system only need to place the column/syringe assembly 46 into the receiving part therefor of the apparatus and to place the comb-like member 50 such that the cannulae 51 thereof extend into the respective solution chambers of a reagent container 64 (one chamber thereof containing the sample to be tested), and to push the start button. The whole test procedure is then automatically effected under the control of the microcomputer 44, the test result being displayed on the display section 45. Thus, the microcomputer 44 will control the step motor 48 and solution distribution means 43 to sequentially aspirate the sample and necessary reagent solutions into the column/syringe assembly 46. The fluorimeter unit 41 will monitor the reaction in the test and reference zones of the column and compare the respective levels obtained. A ratio is calculated, and positive and negative answers are provided on the display 45, depending on whether the result is above or below 1. The computer may, of course, also be programmed to detect any errors of the analytical process or of any of the instrument parts, an error signal then being displayed on the display section 45.

In the following the invention will be illustrated further by means of an example describing the performance of an allergy test in more detail.

Example

A device according to Figs. 1—4 was used, the column tube 9 being of polystyrene and having an inner diameter of 4 mm and a wall thickness of 1.2 mm. The carrier material space confined between filter paper filters 14 and 21 was about 120 μl. The syringe 3 was a conventional 3 ml injection syringe. The detector part of the measuring device comprised a fluorimeter unit and a UV lamp. The measurement was made at an approximate average excitation wave length of 360 nm and an approximate average emission wave length of 450 nm.

Preparation of allergen solution

100 g of pollen (Allergon AB, Vellinge, Sweden) was extracted with 1 liter of phosphate buffer, pH 7.4. The liquid phase was separated off and desalted on a Sephadex® G-25 column (Pharmacia AB, Sweden) equilibrated with 0.1 M NaHCO₃, pH 8.0. Elution was effected with the same buffer and followed on a UV-monitor. Fractions having an absorption at 280 nm were collected and combined.

Coupling of allergen solution to the column matrix

1,43 g of dry CNBr-activated Sepharose® 4B Fast Flow dry gel (Pharmacia AB, Sweden) were swollen for 15 minutes in 50 ml of 1 mM HCl (1.43 g of dry gel is required for 5 ml of swollen gel). The gel was washed with 1 mM HCl on a glass-filter No. 3 (200 ml/g gel) in small portions. One part of gel and two parts of allergen solution were incubated for 2 hours with vertical rotation at room temperature, or over night with vertical rotation at +4°C. The coupling reaction was interrupted by centrifugation at 3—4000 rpm for 15 minutes, and the coupling solution was discarded.

The gel was washed twice with two parts of coupling buffer (0.1 M NaHCO₃, pH 8.3, with 0.5 M NaCl) by mixing and vertical rotation for 5 minutes. After centrifugation the coupling buffer was aspirated and the wash procedure repeated. After the second wash step the gel was incubated with two parts of 1 M ethanolamine, pH 8.0, over night with vertical rotation at +8°C.

The inactivation was interrupted by washing twice with four parts of coupling buffer on a glass-filter No. 2. The gel was washed three times as follows. Four parts of 0.1 M acetate buffer with 1 M NaCl, pH. 4.0, and four parts of 0.1 M NaHCO₃ with 1 M NaHCO₃ with 1 M NaCl, pH 8.3, were aspirated through the gel in small portions on a glass-filter No. 3. A final wash was performed with 0.1 M NaHCO₃ with 1 M NaCl, pH 8.3. The gel can be stored in RAST® buffer No. 3 (Pharmacia AB, Sweden) at +4°C.

Coupling of IgE to the column matrix

Myeloma IgE (prepared according to the process described in U.S. patent 3,720,760) was coupled to CNBr-activated Sepharose® 4B Fast Flow (Pharmacia AB, Sweden) in the same way as above. The selected amount of IgE corresponds to the cut-off concentration.

Packing of column

Allergen-Sepharose® and IgE-Sepharose® prepared as above were packed in one zone each of the column tube 9 between the filters 21 and 14 (Fig. 2), the two zones being separated by the filter 22 (filter paper).

Preparation of sample

A blood sample was taken from a patient and centrifuged in a 400 μl centrifuge tube for about 60 seconds at 10.000 rpm, whereupon the plasma was withdrawn.

Analytical solutions

a) Washing solution:

| | | |
|---|---|---|
| NaCl | 58.5 g | 1.0 M |
| Tween® 20 | 10 ml | 1.0% |
| Bacteriostat, e.g. | | 0.15% |
| Kathon® (Rohm & Haas, | | |
| West Germany) | | |
| $H_2O$ added to 1000 ml | | |

b) Enzyme conjugate:

Anti-IgE-β-galactosidase conjugate from Phadezym® RAST (Pharmacia AB, Sweden) and dissolved in the buffer:

| | | |
|---|---|---|
| $Na_2HPO_4 \times 2H_2O$ | 16.57 g | 0.1 M |
| $NaH_2PO_4 \times H_2O$ | 1.89 g | 2 mM |
| $MgCl_2 \times 6H_2O$ | 0.48 g | 0.2% |
| HSA | 2.11 g | 0.1 M |
| NaCl | 6.16 g | 0.15% |
| Bacteriostat, e.g. | | |
| Kathon® (Rohm & Haas, | | |
| West Germany) | | |
| Sucrose | 52.6 g | |
| $H_2O$ added to 1000 ml | | |

c) Substrate:

0.3 mM 4-methylumbelliferyl-β-D-galactoside in P-buffer, pH 7.4, prepared by adding 0.010 g of 4-methylumbelliferyl-β-D-galactoside to 100 ml of P-buffer:

| | | |
|---|---|---|
| $Na_2HPO_4 \times 2H_2O$ | 7.6 g | 0.5 M |
| $KH_2PO_4$ | 1.0 g | |
| NaCl | 9.0 g | 0.9% |
| $MgCl_2 \times 6H_2O$ | 0.2 g | 0.02% |
| Bacteriostat, e.g. | | 0.15% |
| Kathon® (Rohm & Haas, | | |
| West Germany) | | |
| $H_2O$ added to 1000 ml | | |

Test procedure Test for allergen specific IgE

1. 100 µl of patient serum is aspirated into the column.

2. After 1 minute 0.6 ml of the washing solution is aspirated through the column.

3. 100 µl of enzyme conjugate is aspirated into the column and incubated for 15 minutes.

4. 2 ml of the washing solution is aspirated through the column.

5. 200 µl of substrate is aspirated into the column.

6. The column is placed into the measuring device which indicates if the sample is positive or negative.

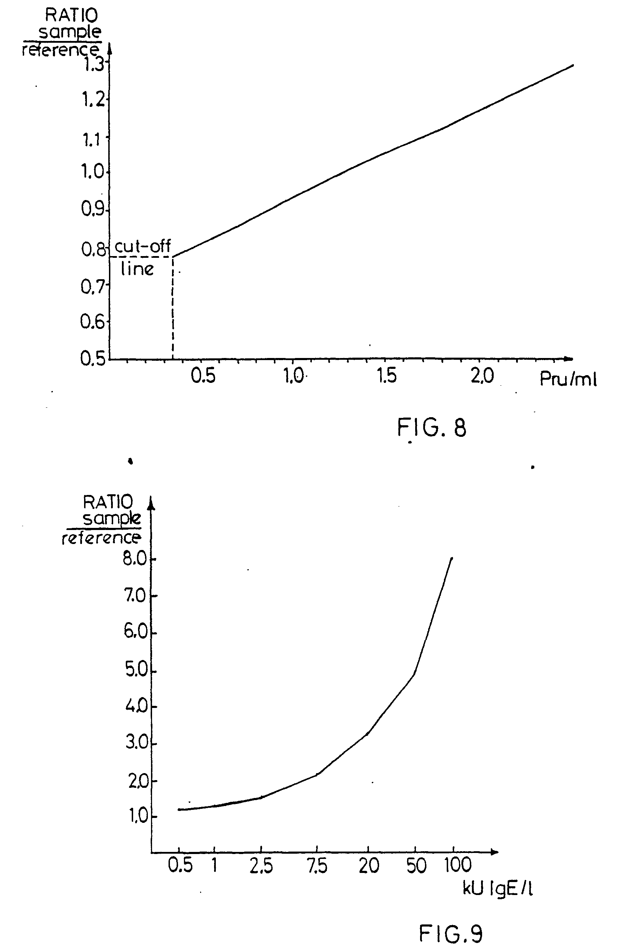

Fig. 8 shows a graph obtained by performing the above test for a number of samples and plotting the ratio of the fluorimeter readings obtained for the sample and reference, respectively, against the IgE contents of the samples as determined by the conventional Phadebas RAST® test (Pharmacia AB, Sweden). Also a cut-off line is indicated.

Total IgE

The column is prepared in the same way as above except that the allergen coupled material is replaced by CNBr-activated Sepharose®-4B Fast Flow, to which anti-IgE has been coupled in the same way as above. Then one proceeds in the same way as in points 1—6 above.

In the above described manner one obtains in a considerably simpler and more rapid way as reliable results as with the conventional Phadebas RAST® test, which i.a. requires incubation over night.

Fig. 9 shows a corresponding graph as Fig. 8 for total IgE (however in semilogarithmic form).

The invention is, of course, not restricted to the above described embodiments thereof, but many modifications and variations may be made without departing from the inventive concept as defined by the accompanying claims.

**Claims**

1. A method for conducting assays of one or more substances of a fluid sample, which assays are based upon biospecific affinity reactions and comprise the binding of said substance or substances to receptors immobilized to a carrier material to form immobilized complexes, and the determination thereof, characterized by providing said carrier material in a column element, wherein the carrier material is divided into at least one test portion to which one or more receptors for the substance or substances to be determined are bound, and at least one reference portion adapted to provide a reference or standard for the substance or substances to be determined, said test and reference portions being arranged either in a superposed relationship in the flow direction or in a parallel relationship, and, to effect said assay reactions, sequentially, and when necessary with intervening incubation, drawing the respective analytical solutions into the column, such that a subsequent addition of solution displaces the preceding one, and then performing said determination directly on the column by a comparative visual or instrumental determination of said test and reference portions.

2. The method of claim 1, characterized in that said at least one reference portion contains a predetermined quantity of the substance or substances to be determined, or of a substance having a corresponding reaction specificity, said substance or substances being covalently bound or immobilized to said carrier material.

3. The method of claim 1 or 2, characterized in that it comprises reacting said carrier-immobilized complex with a marker solution to form a marker labelled complex, which complex, when necessary after a developing reaction, is detected directly or indirectly in said determination.

4. The method of claim 1, 2 or 3, characterized in that said at least one test and reference portions of the column material are superposed layers thereof separated by a filter member or by a layer of non-coupled carrier material.

5. The method of any one of claims 1 to 4, characterized in that said carrier material is particulate agarose, preferably having a particle size of 1—200 micrometers.

6. The method of any one of claims 1 to 5, characterized by testing for allergy by determining immunoglobulin E, the carrier material of said at least one test portion of the column supporting one or more different allergens and said at least one reference portion of the column supporting a predetermined quantity of immunoglobulin E.

7. An apparatus for carrying out the method of claim 1, characterized in that it comprises

a column member (9) having an inlet opening (11) at one end thereof and containing a carrier material, said carrier material being divided into at least one test portion, to which one or more receptors for the substance or substances to be determined are bound, and at least one reference portion, adapted to provide a reference or standard for the substance or substances to be determined, said test and reference portions being arranged either in a superposed relationship in the flow direction or in a parallel relationship; and

an aspirator and fluid collecting device (3, 4) connected or connectable to the other end of said column member (9) and comprising a hollow member (3) having an axially movable plunger means (3a) therein, at least part of said column member (9) being transparent to permit a comparative visual or instrumental determination of the material of said test and reference portions directly on the column.

8. An apparatus according to claim 7, characterized in that said aspirator and fluid collecting device (3, 4) comprises the plunger and cylinder part of an injection syringe (3).

9. An apparatus according to claim 7 or 8, characterized in that said aspirator and fluid collecting device (3, 4) is manually operatable.

10. An apparatus according to any one of claims 7 to 9, characterized in that it comprises a gear wheel and rack assembly (26, 27) for moving said plunger.

11. A system for conducting biochemical assays of substances of fluid samples, characterized in that it comprises the apparatus of any one of claims 7—10 and photometric measuring means (36:41) having a measuring chamber for receiving the column member (9) of said apparatus, said measuring chamber comprising respective measuring sections corresponding to said test and reference portions of the column.

12. A system according to claim 11, characterized in that it further comprises plunger drive means (42) for operative engagement with said apparatus, fluid distribution means (43) for connection to said apparatus, and control means (44) for controlling one or more of said photometric means (36; 41), plunger drive means (42) and fluid distribution means (43).

13. A system according to claim 11 or 12, characterized in that said control means (44) comprise a microcomputer.

**Patentansprüche**

1. Verfahren zur Durchführung von Testverfahren auf eine oder mehrere Substanzen einer flüssigen Probe, wobei die Testverfahren auf biospezifischen Affinitätsreaktionen beruhen und wobei die genannte Substanz oder die Substanzen an auf einem Trägermaterial immobilisierte Rezeptoren gebunden werden, um immobilisierte Komplexe zu bilden, und sie bestimmt werden, dadurch gekennzeichnet, daß das genannte Trägermaterial in einem Säulenelement zur Verfügung gestellt wird, in dem das Trägermaterial in mindestens einen Testteil, an dem ein oder mehrere Rezeptor(en) für die zu bestimmende Substanz oder Substanzen gebunden ist bzw. sind, und mindestens einen Referenzteil, der dazu geeignet ist, eine Referenz oder einen Standard für die zu bestimmende Substanz oder Substanzen zu ergeben, geteilt ist, wobei die genannten Test- und Referenzteile entweder übereinander in Flußrichtung oder parallel angeordnet sind, und daß zur Durchführung der genannten Testverfahren nacheinander und, sofern notwendig, unter Inkubation dazwischen, die jeweiligen Analysen-Lösungen in die Säule eingezogen werden, so daß eine nachfolgende Zugabe von Lösung die vorausgehende ersetzt, und daß dann die genannte Bestimmung direkt auf der Säule durch vergleichende visuelle oder instrumentelle Bestimmung der genannten Test- und Referenzteile durchgeführt wird.

2. Verfahren nach Anspruch 1, dadurch gekennzeichnet, daß mindestens ein Referenzteil eine vorherbestimmte Menge der zu bestimmenden Substanz oder Substanzen oder einer Substanz mit einer entsprechenden Reaktionsspezifität enthält, wobei die genannte Substanz oder Substanzen kovalent an das genannte Trägermaterial gebunden werden oder auf ihm immobilisiert werden.

3. Verfahren nach Anspruch 1 oder 2, dadurch gekennzeichnet, daß der genannte, auf dem Träger immobilisierte Komplex mit einer Marker-Lösung umgesetzt wird, um einen durch einen Marker markierten Komplex zu bilden, wobei der Komplex, sofern notwendig, nach einer Entwicklungsreaktion direkt oder indirekt bei der genannten Bestimmung detektiert wird.

4. Verfahren nach Anspruch 1, 2 oder 3, dadurch gekennzeichnet, daß mindestens ein Test- und Referenzteil des Säulenmaterials als übereinanderliegende Schichten vorliegt und durch ein Filterelement oder durch eine Schicht aus nichtgekoppeltem Trägermaterial getrennt wird.

5. Verfahren nach einem der Ansprüche 1 bis 4, dadurch gekennzeichnet, daß das genannte Trägermaterial teilchenförmige Agarose, vorzugsweise mit einer Teilchengröße von 1 bis 200 μm, ist.

6. Verfahren nach einem der Ansprüche 1 bis 5, dadurch gekennzeichnet, daß beim Allergietest durch Immunoglobulin-E-Bestimmung das Trägermaterial von mindestens einem Testteil der Säule ein oder mehrere verschiedene Allergene trägt und daß mindestens ein Referenzteil der Säule eine vorher bestimmte Menge Immunoglobulin E trägt.

7. Vorrichtung zur Durchführung des Verfahrens nach Anspruch 1, gekennzeichnet durch ein Säulenelement (9), das eine Einlaßöffnung (11) an ihrem einen Ende aufweist und ein Trägermaterial enthält, wobei das Trägermaterial in mindestens einen Testteil, an den ein oder mehrere Rezeptor(en) für die zu bestimmende Substanz oder Substanzen gebunden ist bzw. sind, und mindestens einen Referenzteil, der dazu geeignet ist, eine Referenz oder einen Standard für die zu bestimmende Substanz oder Substanzen zu ergeben, aufgeteilt ist, wobei die genannten Test- und Referenzteile entweder übereinander in Flußrichtung oder parallel angeordnet sind; und

eine Saug- und Flüssigkeitssammelvorrichtung (3, 4), die mit dem anderen Ende des genannten Säulenelements (9) verknüpft oder verknüpfbar ist und einen Hohlkörper (3) mit einem axial beweglichen Kolben (3a) darin umfaßt, wobei mindestens ein Teil des genannten Säulenelements (9) transparent ist, um eine vergleichende visuelle oder instrumentelle Bestimmung des Materials der genannten Test- und Referenzteile direkt auf der Säule zu gestatten.

8. Vorrichtung nach Anspruch 7, dadurch gekennzeichnet, daß die genannte Saug- und Flüssigkeitssammelvorrichtung (3, 4) den Kolben- und den Zylinderteil einer Injektionsspritze (3) enthält.

9. Vorrichtung nach Anspruch 7 oder 8, dadurch gekennzeichnet, daß die genannte Saug- und Flüssigkeitssammelvorrichtung (3, 4) manuell betrieben werden kann.

10. Vorrichtung nach einem der Ansprüche 7 bis 9, dadurch gekennzeichnet, daß sie ein Getriebezahnrad und einen Gestelleinschub (26, 27) zur Bewegung des genannten Kolbens enthält.

11. System zur Durchführung biochemischer Testverfahren auf Substanzen aus flüssigen Proben, dadurch gekennzeichnet, daß es die Vorrichtung nach einem der Ansprüche 7 bis 10 und eine photometrische Meßeinheit (36; 41) mit einer Meßkammer zum Aufnehmen des Säulenelements (9) der genannten Vorrichtung enthält, wobei die genannte Meßkammer entsprechende Meßunterteilungen enthält, die den genannten Test- und Referenzteilen der Säule entsprechen.

12. System nach Anspruch 11, dadurch gekennzeichnet, daß es ferner eine Vorrichtung zum Bewegen des Kolbens (42) zu betriebsbereiten Verknüpfung mit der genannten Vorrichtung, eine Vorrichtung zum Verteilen von Flüssigkeit (43) zur Verbindung mit der genannten Vorrichtung und eine Kontrollvorrichtung (44) zur Kontrolle eines oder mehrerer der genannten photometrischen Einheiten (36; 41) der Vorrichtung zum Bewegen des Kolbens (42) und der Vorrichtung zur Verteilung der Flüssigkeit (43) enthält.

13. System nach Anspruch 11 oder 12, dadurch gekennzeichnet, daß die genannte Kontrollvorrichtung (44) einen Mikrocomputer enthält.

**Revendications**

1. Procédé pour effectuer des essais d'une ou de plusieurs substances d'un échantillon de fluide, lesquels essais reposent sur des réactions d'affinité biospécifique et comprennent la fixation de cette ou de ces substances sur des récepteurs immobilisés sur un matériau de support pour former des complexes immobilisés et leur détermination, caractérisé en ce qu'on fournit ce matériau de support dans un élément en colonne, dans lequel le matériau de support est divisé en au moins une partie test sur laquelle sont fixés un ou plusieurs récepteurs pour la ou les substances à déterminer, et au moins une partie témoin adaptée pour servir de référence ou de standard pour la ou les substances à déterminer, ces parties test et témoin étant disposées soit de façon superposée dans la direction de l'écoulement, soit en parallèle et, pour effectuer ces réactions d'essai, successivement et lorsque que cela est nécessaire avec une incubation intermédiaire, on aspire les solutions analytiques respectives dans la colonne, de telle sorte qu'une addition ultérieure de solution déplace la précédente et ensuite on réalise cette détermination directement sur la colonne par une détermination visuelle ou instrumentale par comparaison de ces parties test et témoin.

2. Procédé suivant la revendication 1, caractérisé en ce qu'au moins une partie témoin contient une quantité prédéterminée de la ou des substances à déterminer ou d'une substance ayant une spéficité réactionnelle correspondante, cette ou ces substances étant fixées de façon covalente ou immobilisées sur ce matériau de support.

3. Procédé suivant la revendication 1 ou 2, caractérisé en ce qu'il comprend la réaction de ce complexe immobilisé sur le support avec une solution de marqueur pour former un complexe marqué par une marqueur, lequel complexe, si cela est nécessaire après une réaction de développement, est détecté directement ou indirectement dans cette détermination.

4. Procédé suivant la revendication 1, 2 ou 3, caractérisé en ce qu'une partie test et une partie témoin au moins présentes du matériau de la colonne sont des couches superposées de celui-ci séparées par un élément de filtration ou par une couche de matériau de support non couplé.

5. Procédé suivant l'une quelconque des revendications 1 à 4, caractérisé en ce que ce matériau de support est de l'agarose particulaire, ayant de préférence une dimension particulaire de 1—200 µm.

6. Procédé suivant l'une quelconque des revendications 1 à 5, caractérisé en ce que l'on recherche une allergie par détermination de l'immunoglobuline E, le matériau de support de cette partie test au moins présente de la colonne portant un ou plusieurs allergènes différents et cette partie témoin au moins présente de la colonne portant une quantité prédéterminée d'immunoglobuline E.

7. Appareil pour effectuer le procédé suivant la revendication 1, caractérisé en ce qu'il comprend

un élément en colonne (9) ayant une ouverture d'entrée (11) à une extrémité et contenant un matériau de support, ce matériau de support étant divisé en au moins une partie test sur laquelle sont fixés un ou plusieurs récepteurs pour la ou les substances à déterminer, et au moins une partie témoin adaptée pour fournir une référence ou un standard pour la substance ou les substances à déterminer, ces parties test et témoin étant disposées soit de façon superposée dans la direction de l'écoulement, soit en parallèle; et

un dispositif (3, 4) aspirateur et collecteur de fluide raccordé ou raccordable à l'autre extrémité de cet élément en colonne (9) et comprenant un élément creux (3) dans lequel se trouve un plongeur (3a) pouvant se déplacer axialement, une partie au moins de cet élément en colonne (9) étant transparent pour permettre une détermination par comparaison visuelle ou instrumentale de la matière de ces parties test et témoin directement sur la colonne.

8. Appareil suivant la revendication 7, caractérisé en ce que ce dispositif (3, 4) aspirateur et collecteur de fluide comprend le plongeur et la partie cylindrique d'une seringue à injection (3).

9. Appareil suivant la revendication 7 ou 8, caractérisé en ce que ce dispositif (3, 4) aspirateur et collecteur de fluide peut être manipulé à la main.

10. Appareil suivant l'une quelconque des revendications 7 à 9, caractérisé en ce qu'il comprend un assemblage de roue d'engrenage et de crémaillère (26, 27) pour déplacer ce plongeur.

11. Système pour effectuer des essais biochimiques de substances dans des échantillons de fluide, caractérisé en ce qu'il comprend l'appareil suivant l'une quelconque des revendications 7 à 10 et des moyens de mesure photométriques (36, 41) ayant une chambre de mesure pour recevoir l'élément en colonne (9) de cet appareil, cette chambre de mesure comprenant des sections de mesure respectives correspondant à ces parties test et témoin de la colonne.

12. Système suivant la revendication 11, caractérisé en ce qu'il comprend en plus des moyens d'entraînement du plongeur (42) pour le faire fonctionner dans cet appareil, des moyens pour la distribution de fluide (43) à raccorder à cet appareil et des moyens de commande (44) pour contrôler l'un ou plusieurs de ces moyens photométriques (36; 41), de ces moyens d'entraînement du plongeur (42) et de ces moyens de distribution du fluide (43).

13. Système suivant la revendication 11 ou 12, caractérisé en ce que ces moyens de commande (44) comprennent un microordinateur.

EP  0 204 807  B1

Fig. 1

Fig. 2

Fig. 3

Fig. 4

1

Fig.5

FIG.6

POSITIVE
NEGATIVE
ERROR

FIG.7

FIG. 8

FIG. 9